(19) **Europäisches Patentamt / European Patent Office / Office européen des brevets**

(11) **EP 4 101 436 A1**

(12) **EUROPEAN PATENT APPLICATION**

(43) Date of publication:
**14.12.2022 Bulletin 2022/50**

(21) Application number: **21179033.2**

(22) Date of filing: **11.06.2021**

(51) International Patent Classification (IPC):
*A61K 8/04* (2006.01)    *A61K 8/34* (2006.01)
*A61K 8/37* (2006.01)    *A61K 8/60* (2006.01)
*A61K 8/92* (2006.01)    *A61Q 19/00* (2006.01)
*B05D 1/00* (2006.01)

(52) Cooperative Patent Classification (CPC):
**A61K 8/046; A61K 8/34; A61K 8/37; A61K 8/375;
A61K 8/602; A61K 8/922; A61Q 19/00;**
A61K 2800/87; B05D 1/04

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **IONIQ Skincare GmbH & Co. KG
88677 Markdorf (DE)**

(72) Inventors:
• **HARNISCH, Eva
88214 Ravensburg (DE)**

• **Verdicchi, Charlotte
22927 Großhansdorf (DE)**
• **LUECK, Annabella
74906 Bad Rappenau (DE)**
• **KRAMER, Carlotta
46325 Borken (DE)**
• **WEMHEUER, Niklas
88690 Uhldingen-Muehlhofen (DE)**

(74) Representative: **Lederer & Keller Patentanwälte
Partnerschaft mbB
Unsöldstraße 2
80538 München (DE)**

(54) **ELECTROSTATICALLY SPRAYABLE SKIN CARING FORMULATION**

(57) The present invention relates to an electrostatically sprayable cosmetic formulation, which can be easily applied on the skin surface of a subject and has a sufficient skin caring effect.

EP 4 101 436 A1

**Description**

The present invention relates to an electrostatically sprayable cosmetic formulation, which can be easily applied on the skin surface of a subject and has a sufficient skin caring effect.

A youthful and vital appearance is a desirable goal for many. This includes a smooth and healthy looking skin. Therefore, numerous cosmetic formulations have been developed to protect the skin from moisture loss. Many of such skin caring products come in form of an oil, creme, lotion or gel, whereby most of the products are crèmes or lotions. Thereby, it is desirable that the formulation is easy and even to apply, absorbs quickly into the skin and leaves the skin feeling comfortable.

However, the number of methods for applying the cosmetic formulation, which is a creme or lotion, on the surface of the skin is limited. The simple method is to apply the creme or lotion by hand. This method includes many disadvantages. In particular, the method does not ensure that a sufficient amount of the lotion is applied on the surface of the skin and in particular this application method does not provide a sufficient coverage of the skin. Moreover, in some cases unnecessary high amounts of the formulation are applied on skin surface that may result into uncomfortable sticky feeling.

Another application method, which should ensure that a sufficient amount of the formulation is applied on the surface of the skin, but in an adequate manner, is the use of pump sprays or pre-pressurized aerosol containers so as to have the formulation atomized and sprayed with the aid of propellant gas. However, conventional aerosol sprays frequently employ volatile compounds as propellants, which are environmentally unfriendly, possible hazardous to health and indeed are being legislated against in many countries. Moreover, it is hardly possible to generate a constant and homogenous flow of the formulation. Therefore, also with this application method a sufficient coverage of the skin surface, i.e. a nearly 100% coverage, is not possible.

In the last few years in the field of cosmetic products a further application method has become of interest. In this application method the cosmetic product is applied on the subject by electrostatic spraying. Such a method is for example described in WO 94/11119 or WO 2001/012139.

This application method can be carried out without the aid of environmentally unfriendly propellants and provides a constant and uniform flow of the product. Hence, it is possible to apply a sufficient amount of the cosmetic formulation on for example the surface of the skin and thus a nearly full coverage of the skin may be possible.

In the prior art different electrostatic spraying devices are known, for example in WO 94/11119, US 2010/0116897 or DE 10 2017 108 610.2 such devices are described. In an electrostatic spaying device a voltage generator creates a high voltage which electrically charges the compounds of the cosmetic product. This results into a spray of the cosmetic product.

However, in this regard it has to be considered that in order to provide a sufficient skin caring effect, the cosmetic formulations contain oil components of synthetic or plant (natural) origin that show a poor electrostatic sprayability. In order to improve the electrostatic sprayability of the formulation additional components may be added to the cosmetic formulations. In doing so, it has to be ensure that these additional components do not diminish the skin caring effect. A requirement, which cannot be easily achieved. For example, as described in EP 3 795 137, hereby incorporated as reference, ethanol is suitable for improving electrostatic sprayability of cosmetic formulations, however, the use of high amounts of ethanol in cosmetic formulations bears the risk that the skin desiccates and thus, it is preferred that the amount of ethanol present in a skin caring formulation is as low as possible. Furthermore, the use of alcohol even in high quantities in a cosmetic formulation does not ensure good electrostatic sprayability of the cosmetic formulation in every case.

Additionally, it is known that even though some of the cosmetic formulations have appropriate electrical characteristics, it is still not possible to electrostatically spraying these formulations such that all parts of the skin are covered by the cosmetic formulation. This result into a socalled streaking effect on the skin, i.e. the applied cosmetic formulation creates a "zebra crossing" pattern on the skin.

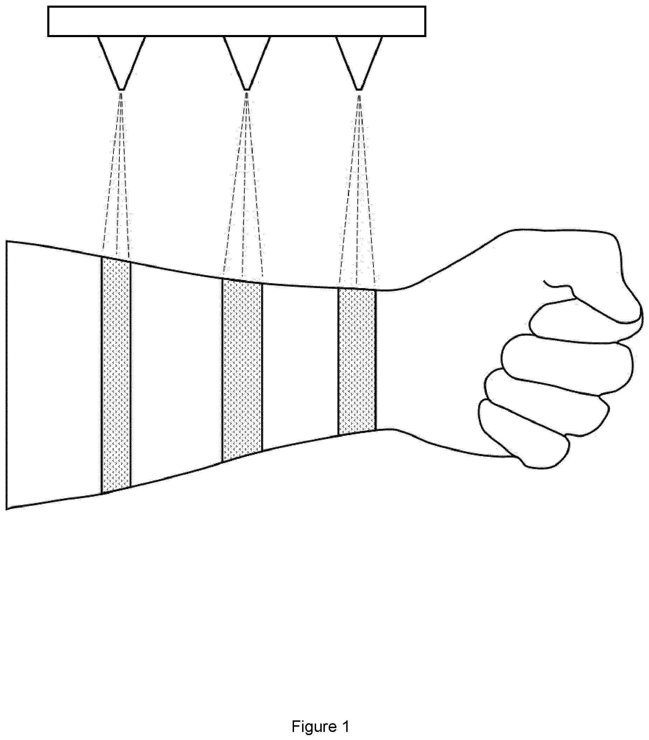

Figure 1 schematically shows the undesired "zebra crossing" pattern on the skin, which occurs if the cosmetic formulation is not uniformly sprayable and thus, uneven distribution of the formulation on the skin is possible.

However, a non-homogeneous distribution of the formulation on the skin leads to the fact that not all skin areas receive sufficient amounts of the care product or, in the case of others, the amounts of lotion applied are too high, which results in an uncomfortable skin feeling. In any case, the skin caring effect is not optimal.

Additionally, many cosmetic formulations, in particular skin caring products, are based on emulsions. These emulsions can be water-in-oil-emulsions, oil-in-water-emulsions, alcohol-in-oil-emulsions, oil-in-alcohol emulsions, or oil-in-oil-emulsions. In general, an emulsion is a thermodynamically unstable system consisting of at least two immiscible liquid phase one of which is dispersed as droplets/globules in other liquid phase. The instability of emulsions leads to floating of droplets, cohesion between droplets, and finally to creaming and separation and thus, usually, the electrostatic sprayability of emulsions is poor.

In order to improve the stability of emulsions surfactants are used generally. Surfactants are substances that decrease the surface tension between the two immiscible liquid phases and thus improves the stability of the emulsion. Many various surfactants that are suitable for cosmetic formulations are known in the art. Surfactant are classified in anionic, cationic, amphoteric and non-ionic surfactant. Examples for surfactants are: glyceryl esters of fatty acids, polyethylene glycol esters of fatty acids and alkoxylated derivatives thereof, sodium laurate sulfate, sulfated castor oil, sodium coco-sulfate, sodium cocoyl-glutamate, glycolipids, quaternary ammonium compounds such as methyl triethanolammonium, coco-glucoside, lauryl glucoside, polygylceryl-4-caprate, cocamidopropyl betaine, capryl/capramidopropyl betaine, polysorbats, etc.

However, even though surfactants decrease the surface tension of the components present in the formulation, as demonstrated in the examples below, the decrease of the surface tensions of the components present in the cosmetic formulation does not result in an improved electrostatic sprayability of the cosmetic formulation. On the contrary, also the electrostatic sprayability of such a cosmetic formulation is not sufficient.

Hence, object of the application was to provide an electrostatically sprayable cosmetic formulation having an improved sprayability by maintaining its good skin caring effect.

It has been surprisingly found that the electrostatic sprayability of the cosmetic formulation can be improved, if the cosmetic formulation comprises in addition to a combination of ethanol, a polar oil component and a further oil component that differs from the polar oil component and which is chosen from components of plant origin a glycolipid as surfactant, i.e. a lipid with a carbohydrate attached by a glycoside (covalent) bond. In that case, the amount of ethanol present in the formulation is in a range, which does not diminish the skin caring effect of the cosmetic formulation.

Hence, the present invention provides an electrostatically sprayable cosmetic formulation, comprising

a) 10 to 30 wt.-% ethanol;
b) 50 to 80 wt.-% of at least one polar oil component chosen from emollients, which are liquid at 20 °C and have a log $K_{ow}$ of 2 to 10;
c) 5 to 30 wt.-% of a further oil component different from the emollients constituting the polar oil component and chosen from oils of plant origin; and
d) 0.5 to 3 wt.-% of a glycolipid surfactant;
based on the total weight of the formulation, and wherein the sum of the weight of all components present in the cosmetic formulation is 100 %.

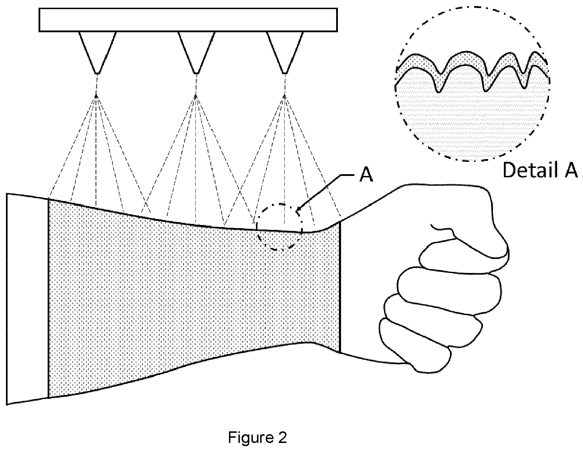

Figure 2 schematically shows the full coverage of the skin by using a cosmetic formulation of the invention, i.e. no streaking effect occurs. The enlarged section "Detail A" of Figure 2 shows a part of the skin, which is covered with the cosmetic formulation according to the invention.

The cosmetic formulation of the invention is a homogenous stable (transparent) solution, i.e. all components are completely dissolved in the formulation. In other words, there is no formation of flakes or crystals due to insufficiently dissolved components in the formulation, which may negatively influence the properties, in particular the electrostatic sprayability, of the cosmetic formulation.

The abbreviation "wt.-%" or "w/w", as used herein, means "weight percentage" and refers to the weight amount of a compound in relation to the (total) weight of a formulation of compounds or of a substrate if nothing else is explicitly stated or obvious under the circumstances.

The term "comprising", "including" or "containing" includes "consisting essentially of" and also "consisting of".

In the present specification, the use of "a" in the singular also comprises the plural ("some"), and vice versa, unless the context clearly indicates the contrary.

The electrostatically sprayable cosmetic formulation having the specific combination of components according to the invention preferably shows an specific electrical resistance of 15 to 100 GOhm·mm²/m, more preferably of 20 to 85 GOhm·mm²/m, it is even more preferred that the specific electrical resistance is not higher than 80, not higher than 60, not higher than 50 GOhm·mm²/m, most preferably the electrostatic resistance is between 35 to 48 GOhm·mm²/m, determined as described below (see measurement methods). This ensures that the formulation is good electrostatically sprayable and thus no undesired streaking effect on the skin occurs.

In addition, in order to provide a cosmetic formulation which shows a good electrostatic spraying behavior, the formulation preferably has a surface tension of 15 to 50 mN/m, more preferably of 20 to 45 mN/m, 22 to 40 mN/m, more preferably 24 to 35 mN/m determined by the stamp method as described below (see measurement methods).

Furthermore, the relative permittivity of the cosmetic formulation according to the invention may be at least 1.0, preferably at least 1.5. In particular, the relative permittivity of cosmetic formulation preferably is between 1.2 and 5.0, more preferably between 1.5 and 3.0, or between 1.7 and 2.0. The measurement method for determining the relative permittivity according to the invention is described below (see measurement methods).

Moreover, the cosmetic formulation of the invention may have a viscosity of from 0.65 mPa·s to 5000 mPa·s, preferably of from 1.0 to 1000 mPa·s, 5.0 to 500 mPa·s, more preferably of from 10.0 to 100 mPa*s, determined as described below (see measurement methods). In particular a good spraying behavior of the cosmetic formulation can be observed if the cosmetic formulation has a viscosity below 4.0 mPa·s. A cosmetic formulation having a viscosity of at least 8.0 mPa·s shows a sufficient spraying behavior.

For spaying the cosmetic formulation on the skin surface of a subject, every electrostatic device known in the art can be used. However, it is preferred to use a device as described in DE 10 2017 108 610.2, which is further specified in DE 10 2017 108 612.9, DE 10 2017 108 613.7, DE 10 2107 108 614.5 and DE 10 2107 108 615.3.

As mentioned above, due to the presence of oil components in cosmetic formulations, in particular in skin caring formulations, the electrostatic sprayability of these formulations is poor. According to the invention, in order to ensure that the cosmetic formulation has a sufficient electrostatic sprayability, the cosmetic formulation comprises at least one polar oil component (first oil component) chosen from emollients that have a polarity of log $K_{ow}$ of 2 to 10, and a further oil component, i.e. a second oil component, that differs from the emollients constituting the polar oil component and which is chosen from oils of plant origin.

Emollients, also referred to as cosmetic oils, are used in numerous cosmetic formulations for softening and smoothing the scales of the skin, which help to reduce rough and flaky skin. They are frequently occlusive agents, i.e. substances that provide a layer of protection that help prevent moisture (water) loss from the skin. Examples for emollients are silicones, such as dimethicone or cyclomethicone, stearyl alcohol or cetyl alcohol, and petrolatum derivatives such a petroleum jelly or mineral oil. In general, most of the emollients as used in cosmetic formulation are non-polar and thus due to their electrical characteristics, i.e. resistivity and permittivity, most of the emollients show a poor electrostatic sprayability. However, some of them have a sufficient polarity and thus are interesting for electrostatically sprayable cosmetic formulations.

In the cosmetic field, one possibility to define the polarity of a compound is to determine the n-octanol/water partition coefficient ($K_{ow}$ or P). The partition-coefficient refers to the ratio of concentrations of the compounds in the mixture of these two immiscible phases at equilibrium. Hence, the partition coefficient measures how hydrophilic ("water-loving") or hydrophobic ("water-fearing") the tested compound is. In most of the cases the n-octanol/water partition coefficient is stated as decade logarithms log $K_{ow}$ or log P. The determination of the n-octanol/water partition coefficient is described for example in J. Sangster, "Octanol-Water Partition Coefficients: Fundamentals and Physical Chemistry", Vol. 2 of Wiley Series in solution chemistry, John Wiley & Sons, Chichester, 1997.

According to the invention, the log $K_{ow}$ of the emollients, which are suitable for the cosmetic formulation of the invention, is between 2.0 to 10.0, preferably between 2.5 and 9.5, 3.0 and 8.5, or between 3.5 and 7.5.

Examples which fulfills these requirements are known in the art. In particular, the emollients used in the cosmetic formulation of the invention are preferably selected from the group consisting of octyldodecanol dibutyl adipate, co-cocglyceride, PPG-15 stearyl ether, PPG-3 myristyl ether, PPG-14 butyl ether, dicaprylyl carbonate, isoamyl cocoate, diethylhexyl carbonate, isopropyl myristate, isopropyl palmitate, decyl cocoate, phenoxyethyl caprylate, C12-C15 alkyl benzoate and combinations thereof, more preferred is that the at least one emollient is octyldodecanol (e.g. Eutanol G®), dibutyl adipate (e.g. Cetiol B®) or cocoglyceride (e.g. Myritol 331®, modified coconut oil, CASR-No. 68606-18-8).

It is preferred that the cosmetic formulation of the invention comprises as polar oil component a mixture of at least two or more different emollients, at least three, but not more than five different emollients. In particular, it is preferred that the polar oil component of the cosmetic formulation comprises or is a mixture of octyldodecanol, dibutyl adipate and cocoglyceride.

The different emollients of the cosmetic formulation are used in different or equal amounts in the formulation so that the total amount of emollients constituting the polar oil component is between 50 to 80 wt.-%, based on the total weight of the formulation. Preferably, the total amount of emollients present in the formulation is from 52 to 75 wt.-%, 55 to 70 wt.-%, more preferably from 57 to 65 wt.-%, based on the total weight of the formulation.

The further (second) oil component present in the cosmetic formulation of the invention differs from the emollients constituting the polar oil component of the cosmetic formulation and is chosen from oils of plant origin, also called natural oils or vegetable oils. Oil components of plant origin, which are suitable for cosmetic formulations, are well known in the art. Examples thereof are hydrogenated palm kernel oil, jojoba oil (*simmondsia chinensis seed oil*), althea officinalis extract, apricot (*prunus armeniaca*) kernel oil, arnica montana extract, birch (*betula alba*) bark extract, borage (*Borago officinalis*) extract, butcherbroom (*ruscus aculeatus*) extract, candelilla (*euphorbia cerifera*) wax, *calendula officinalis* extract, canola oil, cardamon (*elettaria cardamomum*) oil, carnauba (*copernicia cerifera*) wax, carrot (*Daucus carota sativa*) oil, castor oil (*ricinus communis seed oil*), chamomile (*anthemis nobilis*) oil, clary (*salvia sclarea*) oil, cocoa (*Theobroma* cacao) butter, coconut (*Cocos nucifera*) oil, corn (Zea *mays*) oil, eucalyptus globulus oil, evening primrose (*Oenothera biennis*) oil, nut oils for example *macadamia ternifolia seed oil,* olive (*Olea europaea*) oil lavender (*Lavandula angustifolia*) oil, lemon (*citrus medica limonum*) oil, rice (*Oryza sativa*) bran oil, peppermint (*Mentha piperita*) oil, rosemary (*Rosmarinus officinalis*) oil, rose oil, safflower (*carthamus tinctorius*) oil, sage (*salvia officinalis*) oil, sandalwood (*santalum album*) oil, sesame (*Sesamum indicum*) oil, silk powder, sunflower (*Helianthus annuus*) seed oil sweet almond (*prunus amygdalus dulcis*) oil, soybean (*glycine soja*) oil, wheat (*Triticum vulgare*) germ oil, and ylang ylang (*cananga odorata*) oil, *persea gratissima* (avocado) oil, *argania spinosa* (argan) kernel oil, *melaleuca alternifolia* (tea tree) leaf oil etc.

According to the invention, it is preferred that the second oil component is selected from the group of natural oils consisting of *simmondsia chinensis seed oil, macademia ternifolia seed oil, ricinus communis seed oil, prunus amygdalus dulcis* (sweet almond) oil, *persea gratissima* (Avocado) oil, *argania spinosa* (argan) kernel oil, *melaleuca alternifolia* (tea tree) leaf oil and mixtures thereof. In particular it is preferred that the second oil component of the cosmetic formulation of the invention is selected from the group of natural oil consisting of *simmondsia chinensis seed oil, macademia ternifolia seed oil, ricinus communis seed oil,* and mixtures thereof, and more preferred that the second oil component comprises or is a mixture of *chinensis seed oil, macademia ternifolia seed oil* and *ricinus communis seed oil.*

The second oil component is present in an amount of 5 to 30 wt.-%, preferably in amount of 10 to 25 wt.-%, more preferably in amount of 15 to 20 wt.-%, based on the total weight of the formulation, in the cosmetic formulation.

In order to improve the electrostatic sprayability of the oil components present in the cosmetic formulation, the formulation according to the invention additionally comprises ethanol. It has been found that an ethanol amount of 10 to 30 wt.-%, based on the total weight of the composition is sufficient in case the cosmetic formulation contains additionally a glycolipid surfactant. Preferably the amount of ethanol is between 15 and 28 wt.-%, or between 17 and 25 wt.-%, more preferably between 18 and 23 wt.-%, based on the total weight of the formulation.

According to the invention, the surfactant used in the cosmetic formulation has to be a glycolipid in order to improve the electrostatic sprayability of the formulation.

Glycolipids, which are suitable for cosmetic formulations are sophorolipids, rhamnolipids and mannosylerythritol lipids as for example described in the publication of Lourith et al., International Journal of Cosmetic Science, 2009, 31, pages 255-261, hereby incorporated as reference. In particular, it is preferred that the cosmetic formulation of the invention includes a rhamnolipid as surfactant. Rhamnolipids can be produced by *Pseudomonas sp.* or *Pseudomonas aeruginosa* and are commercial available. Rhamnolipids are composed of one or two rhamnose molecules and are bonded to up to three molecules of hydroxyl fatty acids of varying chain length from 8 to 14 carbon atoms of which $\beta$-hydroxydecanoic acid is predominant.

According to the invention, an amount of between 0.5 and 3.0 wt.-% of the glycolipid is sufficient to improve the electrostatic sprayability of the cosmetic formulation. Preferably the amount is between 0.6 and 2.5 wt.-%, 0.7 and 2.0 wt.-%, more preferably between 0.75 and 1.5 wt.-%, based on the total weight of the formulation.

Although the cosmetic formulation comprises oil components, the cosmetic formulation may comprises at least one moisture agent for further improving the skin caring effect of the formulation. Suitable moisture agents are for example fructose, glucose, glycerin, propylene glycol, 1,3-butylene glycol, linolenic acid, hyaluronic acid, beeswax, hydrogenated starch hydrolysate, acetylate lanolin alcohol, ceramides, ceresin, collagen, collagen, algae extract, aloe barbadensis, aloe-barbadensis extract, aloe barbadensis gel, and mixtures thereof. It is preferred that the moisture agent is selected from the group consisting of glycerin, propylene glycol, aloe barbadensis, aloe-barbadensis extract and aloe barbadensis gel and mixtures thereof. In particular, it is preferred that the cosmetic formulation comprises aloe barbadensis leaf extract, more preferably aloe barbadensis leaf extract/isopropyl myristate as moisture agent.

The amount of the moisture agent present in the cosmetic formulation of the invention is preferably of 0.05 up to 2.0 wt.-%, 0.1 up to 1.5 wt.-%, and more preferably 0.8 up to 1.0 wt.-%, based on the total weight of the formulation.

In common, cosmetic formulations comprise in addition to the above described active compounds also cosmetic additives such as cosmetically acceptable carriers, sterols, amino acids powders, colorants, pigments, dyes, pH adjusters, perfumes, antioxidants, vitamins E, pro-vitamins, essential fatty acid, sphingolipids, UV filters etc. and combinations thereof. These additives and combinations thereof may be also present in the cosmetic formulation of the invention.

Preferably, according to the invention, the additives are selected from the group consisting of perfumes, vitamins and/or pro-vitamins such as tocopherol and/or tocopheryl acetate, and combinations thereof. It is particular it is preferred that the formulation includes at least tocopherol as additive.

The cosmetic formulation of the invention preferably comprises 0.2 to 3.0 wt.-% of at least one cosmetic additive, more preferably of 0.5 to 2.5 wt.-%, 1.0 to 2.0 wt.-%, even more preferably of 1.2 to 1.8 wt.-%, based on the total weight of the formulation.

The cosmetic formulation of the invention may include minor amounts of water. Usually, for example surfactant added to the cosmetic formulation in form of aqueous mixtures. These minor amounts of water do no influence negatively the electrostatic sprayability of the formulation. However, the amount of water should be not higher than 5 wt.-%, based on the total weight of the formulation. It is more preferred that the amount of water present in the cosmetic formulation of the invention is less than 5 wt.-%, preferably less than 3.5 wt.-%, less than 2.5 wt.-%, more preferably less than 1 wt.-%, based on the total weight of the formulation. In particular it is preferred that the amount of water present in the cosmetic formulation is between 0.5 and 4.9 wt.-%, between 0.70 wt.-% and 2.0 wt.-%, and more preferred between 0.75 wt.-% and 1.0 wt.-%, based on the total weight of the cosmetic formulation.

Furthermore, due to the good electrostatic sprayability of the cosmetic formulation of the invention, there is no need that the formulation further comprises a propellant in order to ensure that the formulation is sprayable.

In cosmetic and dermatology industry different propellants are used, for example, chemically inert hydrocarbons such as propane, n-butane, isobutene and cyclopropane, and mixtures thereof, as well as halogenated hydrocarbons such as for example dichlorodifluormethane or 1,1-dichloro-1,1,2,2-tetrafluoroethane. isobutane, used singly or mixed with other hydrocarbons, particularly propane is for example preferred used in aerosol. The use of propellants involves numerous disadvantages that can be avoided by providing the formulation of the invention.

The cosmetic formulation of the invention can be used alone or in a mixture comprising other cosmetic formulations. Preferably, the cosmetic formulation is a creme or a lotion, more preferably the cosmetic formulation is a lotion, and even more preferred a body lotion.

In a further embodiment of the invention the electrostatically sprayable cosmetic formulation comprises

a) 15 to 25 wt.-% ethanol;
b) 52 to 65 wt.-% of a mixture of emollients constituting the polar oil component and wherein each emollient is liquid at 20 °C and have a log $K_{ow}$ of 2 to 10;
c) 10 to 20 wt.-% of a mixture of oil components of plant origin, which differ from the emollients, constituting the further oil component;
d) 0.6 to 2.0 wt.-% of a glycolipid surfactant,
e) 0.5 to 1.5 wt.-% of at least one moisture agent,
f) 0.2 to 3.0 wt.-% of at least one cosmetic additive, and
g) less than 5.0 wt.-% of water;
based on the total weight of the formulation, and wherein the sum of the weight of all components present in the cosmetic formulation is 100 %.

In particular, it is preferred that the electrostatically sprayable cosmetic formulation consists of

a) 15 to 25 wt.-% ethanol;
b) 53 to 63 wt.-% of a mixture of emollients constituting the polar oil component and wherein each emollient is liquid at 20 °C and have a log $K_{ow}$ of 2 to 10;
c) 13 to 20 wt.-% of a mixture of oil components of plant origin, which differ from the emollients, constituting the further oil component;
d) 0.75 to 1.5 wt.-% of a glycolipid surfactant,
e) 0.5 to 1.5 wt.-% of at least one moisture agent,
f) 0.2 to 3.0 wt.-% of at least one cosmetic additive, and
g) less than 3.0 wt.-% of water;
based on the total weight of the formulation, and wherein the sum of the weight of all components present in the cosmetic formulation is 100 %.

More preferred is a electrostatically sprayable cosmetic formulation consists of

a) 15 to 25 wt.-% ethanol;
b) 53 to 63 wt.-% of a mixture of emollients constituting the polar oil component and which consists of 20 to 23 wt.-% dibutyl adipate, 8 to 12 wt.-% cocoglyceride and 25 to 28 wt.-% octyldodecanol;
c) 13 to 20 wt.-% of a mixture of oil components of plant origin constituting the further oil component, which differs from the polar oil component and which consists of 4 to 6 wt.-% *simmondsia, chinensis seed oil,* 1.5 to 4.0 wt.-% *macademia ternifolia seed oil* and 7.5 to 10.0 wt.-% *ricinus communis seed oil*;
d) 0.75 to 1.5 wt.-% of rhamnolipid;
e) 0.5 to 1.5 wt.-% of *aloe barbadensis leaf extract*;
f) 1.0 to 2.0 wt.-% of a cosmetic additive mixture, which consists of 0.45 to 0.85 wt.-% perfume, 0.1 to 0.3 wt.-% tocopherol and 0.45 to 0.85 wt.-% tocopherol acetate; and
g) 0.75 to 1.0 wt.-% of water;
based on the total weight of the formulation, and wherein the sum of the weight of all components present in the cosmetic formulation is 100 %.

The examples that follow are intended for illustrating the invention in more detail.

## Examples

### 1. Measurement methods

1.1 Specific Electrical Resistance

The specific electrical resistance was determined by the means of an Electrospray Paint Test Meter of DCA Electronic Ltd. The measuring current was between 85 μA and 100 μA or higher. The measuring range was 10 kOhm up to 5 MOhm. The measured electrical resistance was approximately conversed to the specific electrical resistance according to ASTDM D5682-08.

### 1.2 Surface Tension

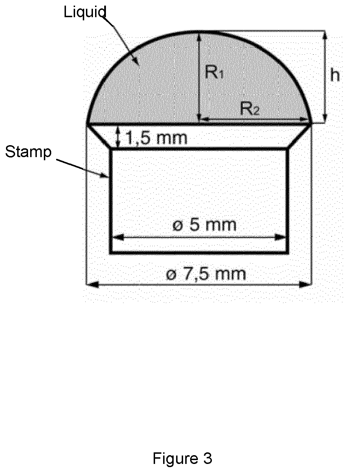

The surface tensions of the formulations as described herein were measured by the stamp method as described below:
The metal stamp can be used to determine the surface tension of liquids and highly viscous media (e.g. polymer melt or adhesives). The liquid is applied to the stamp, which forms an entire surface and a drop on the stamp. The stamp material has no influence on the measurement result.
The surface tension of the liquid can be calculated from the drop contour and the density of the liquid using the SCA 20 software (www.dataphysics-instruments.com, DATAPhysics OCA20) and the Young-Laplace equation (see Figure 3).
In addition to the surface tension, the contact angle and the volume of the drop are also measured. The determined surface tension is independent of the contact angle. The same surface tension values are measured both when the droplet is enlarged and when it is reduced by suction.

### 1.3 Relative Permittivity

The relative permittivity was determined by the means of a High Frequency Liquid Dielectric Constant Meter (Time Domain Reflection) "ALPHA TDR-5000" of Zadow Electronics (ww.zadow-electronics.de) having two parallel wire sensors that have a length of 5 cm. The measurement method was carried out such that the TDR-500 periodically sends voltage pulses with times (250 ps) onto the sensor cable to determine the dielectric constant (K) of the formulations:

$$K = 15^2 \times (\frac{t}{r})^2$$

t = running time in ns; the running time was 5 ns
r = length of the wire sensor (5 cm)

The relative permittivity of the formulation was calculated on the basis of the determined dielectric constant.

### 1.4 Electrical Conductivity

The electrical conductivity was measured by means of Sartorious PT-20, hand-held conductivity meter, measuring cell PY-CL1 (www.sartorius.de). Measurement at room temperature.

### 1.5 Viscosity

The viscosity was determined by means of a Universal Dynamic spectrometer "Physica UDS 200" at shear rates of 1 to 3000 s$^{-1}$ and 22 °C.

### 1.6 Spraying Test

The spraying test was carried out such that the distance from a spraying nozzles to the outer left side of a metal tube was 13 cm. The pre-set voltage of test was 8 kV.
For the fluid pump a voltage of 3.8 V was used. After changing each test liquid, the tube system was rinsed with isopropanol. The spraying behavior of the formulations was optically evaluated and the spraying pattern was assessed to the following scale: good; moderate and poor.

### 1.7 Application Studies

In these studies, it was tested whether the cosmetic formulation can be easily and evenly applied on the skin, absorbs quickly into the skin and leaves a comfortable skin feeling. The cosmetic formulation was applied on the skin by each consumer her/himself over a period of fourteen days by using an IONIQ spraying system, which is described in detail in DE 10 2017 108 610.2, DE 10 2017 108 612.9, DE 10 2017 108 613.7, DE 10 2107 108 614.5 and. DE 10 2107 108 615.3, by using a pre-set voltage of 8 kV. The consumers were interviewed at four predetermined time points regarding their impression of the cosmetic formulation according to the invention with respect to the following issues:

- Effect regarding softness of the skin and skin feeling by using the cosmetic formulation of the invention in comparison to their preferred body lotion;
- Appearance of the cosmetic formulation of the invention on the skin
- Absorption of the cosmetic formulation of the invention into the skin.

## 2. Cosmetic Formulation

All used raw materials are cosmetic qualities and commercially available. The used ethanol was denaturized quality (denaturant: isopropyl and tert.-butanol; Ethanol 96%), the used aloe barbadensis extract was *aloe barbadensis herbasol* extract IPM 201100.02.2 from Lipoid GmbH and the *simmondsia chinensis seed oil* was Ewanol Jo from Wagner, the used *macadamia ternifolia seed oil* was Ewanol MN-R from Wagner and the used *ricinus communis seed oil* was Ewanol Ri-G from Wagner.

The components of each formulation were mixed together, whereby in a first step the oil components were mixed with ethanol to obtain a homogenous and transparent mixture. Afterwards, the further components were added to the mixture.

For testing the influence of surfactant on the spraying behavior of the cosmetic formulation, each surfactant was added to the cosmetic formulation in form of an aqueous surfactant mixture in an amount of 1.5 wt.-%, based on the total weight of the formulation, such that the active matter of the tested surfactants present in the cosmetic formulation was between 0.04 and 0.75 wt.-%, based on the total weight of the cosmetic formulation (see Table 2 below). Particular care was taken to maintain the transparency of the cosmetic formulation, because the transparency of the cosmetic formulation is an indicator for its stability. As mentioned above, the mixture of oil component(s) and ethanol is a transparent and homogenous solution. However, the further components, which are added to the mixture, in particular the surfactant, may be not dissolved properly in the cosmetic formulation. This show up in formation of small flakes, crystals or a cloudiness. As already discussed, the undissolved components have a negative effect on the properties of cosmetic formulation, in particular on the electrostatic sprayability of the cosmetic formulation. Hence, stability in the meaning of the invention means that all components are completely dissolved in the cosmetic formulation which results into a transparency solution. As shown in Table 2 below, not in every case it was possible to obtain a transparent solution.

The electrostatic spraying behavior of the different cosmetic formulations was tested according to spraying test directly after preparation of the formulation.

The composition of the cosmetic formulation without surfactant is given in Table 1 below:

| Material | INCI | Formulation [wt.-%] |
|---|---|---|
| Cetiol B | DIBUTYL ADIPATE | 22.1 |
| Myritol 331 | COCOGLYCERIDES | 9.0 |
| Eutanol G | OCTYLDODECANOL | 26.2 |
| Total amount of emollients (polar oil component) | | 57.3 |
| Ethanol denatured (Isopropanol/ Tert.-butanol) | ALCOHOL DENAT. | 22.0 |
| Ewanol JO (Jojobaoil, cold-pressed DAC) | SIMMONDSIA CHINENSIS SEED OIL | 5.0 |
| Ewanol MN-R (Macadamia nut oil raff. DAC) | MACADAMIA TERNIFOLIA SEED OIL | 3.0 |
| Ewanol RI-G castor oil pressed Ph. Eur. | RICINUS COMMUNIS SEED OIL | 9.0 |
| Total amount of oil components of plant origin (second oil component) | | 17.0 |
| Aloe (Barbadensis) Herbasol Extrakt IPM 201100.02.2 | ALOE BARBADENSIS LEAF EXTRACT, ISOPROPYL MYRISTATE | 1.0 |

| Parfum oil | PARFUM | 0.5 |
|---|---|---|
| DL-alpha-Tocopherol (DSM) | TOCOPHEROL | 0.2 |
| DL-Alpha-Tocopherolacetate (DSM 0420085) | TOCOPHERYL ACETATE | 0.5 |
| total amount of additives | | 1.2 |

Table 1

**3. Results**

3.1 Electrostatic Sprayability

Even though the cosmetic formulation had a sufficient stability and contained ethanol in combination with a mixture of polar emollients and natural oils (see Table 1 above), the electrostatic sprayability of the cosmetic formulation were not sufficient without the use of a surfactant in the formulation.

Additionally, the use of any surfactant in the cosmetic formulation also did not provide the desired improvement of the spraying behavior of the cosmetic formulation. As can be seen from Table 2 below, only the use of a glycolipid, e.g. rhamnolipid, as surfactant provides a transparent solution and additionally an improved spraying result.

| Surfactant [wt.-% active matter] | Description of solution | Spray Result |
|---|---|---|
| **Amphoteric surfactants** | | |
| Cocamidopropyl betaine [0.04] | transparent | poor (streaky) |
| Cocamidopropyl betaine [0.19] | opaque | poor (streaky) |
| Disodium cocoamphodiacetate [0.2] | opaque | moderate (but several larger droplets) |
| Capryl/capramidopropyl betaine [0.18] | opaque | poor (streaky) |
| Coco-betaine [0.15] | opaque | moderate |
| **Anionic surfactants** | | |
| Sodium laureth sulfate [0.14] | transparent | Poor (streaky) |
| Sulfated castor oil [0.25] | transparent | Poor (streaky) |

| | | |
|---|---|---|
| Sodium coco-sulfate [0.15] | transparent | Moderate (several larger drops] |
| Sodium cocoyl glutamate [0.13] | opaque | Moderate (several larger drops) |
| Sodium C14-C16 olefin sulfonate [0.19] | transparent | Moderate (several larger drops] |
| Sodium coco-glucoside tartrate [015%] | opaque | Very poor (streaky) |
| Diethylhexyl sodium sulfosuccinate [0.19] | transparent | Poor (streaky) |
| Rhamnolipid [0.75] | transparent | good |
| **Non-ionic surfactants** | | |
| Coco-glucoside [0.2] | opaque | Poor (streaky) |
| Lauryl glucoside [0.26] | opaque | Poor (streaky) |
| Polyglyceryl-4 caprate [0.23] | transparent | Very poor (streaky) |

Table 2

The cosmetic formulation according to the invention has a specific electrostatic resistance of 63.4 GOhm·mm$^2$/m, a surface tension of 26.74 mN/m, a relative permittivity of 1.99 and viscosity of 9 mPa·s at a shear rate of 100/s.

3.2 Application Studies

In the application studies, no consumer had difficulty to apply the cosmetic formulation of the invention on the skin by using the IONIQ spraying system. Furthermore, in summary, i.e. across all four predetermined interview time points, the consumers rated the cosmetic formulation of the invention as followed:

3.2.1 *Effect regarding softness of the skin and skin feeling*

Approx. 60 % of the consumers confirmed clear advantages of the cosmetic formulation of the invention regarding softness of the skin in comparison to their preferred body-lotion. Approx.
85 % of the consumers confirmed a comfortable feeling after application of the cosmetic formulation.

3.2.2 *Appearance of the cosmetic formulation on the skin*

85% of consumers were of the opinion that the appearance of the cosmetic formulation on the skin is (very) appeal. 68% of the consumers thought that the formulation is shiny on the skin and 54% confirmed that the cosmetic formulation is invisible on the skin.

3.2.3 *Absorption of the cosmetic formulation into the skin*

72% of the consumers confirmed that the cosmetic formulation of the invention is absorbed (very) quickly and completely into the skin.

**Claims**

1. Electrostatically sprayable cosmetic formulation, comprising

   a) 10 to 30 wt.-% ethanol;
   b) 50 to 80 wt.-% of at least one polar oil component chosen from emollients, which are liquid at 20 °C and have a log $K_{ow}$ of 2 to 10;
   c) 5 to 30 wt.-% of a further oil component different from the emollients constituting the polar oil component and chosen from oils of plant origin; and
   d) 0.5 to 3 wt.-% of a glycolipid surfactant;

   based on the total weight of the formulation, and wherein the sum of the weight of all components present in the cosmetic formulation is 100 %.

2. Electrostatically sprayable cosmetic formulation according to claim 1, further comprising at least one moisture agent in an amount of 0.05 to 2 wt.-%, based on the total weight of the formulation.

3. Electrostatically sprayable cosmetic formulation according to claims 1 and 2, further comprising at least one cosmetic additive in an amount of 0.2 to 3 wt.-%, based on the total weight of the formulation.

4. Electrostatically sprayable cosmetic formulation according to any one of the previous claims, wherein the emollient constituting the polar oil component is selected from the group consisting of dibutyl adipate, cocoglyceride, octyldodecanol, and mixtures thereof.

5. Electrostatically sprayable cosmetic formulation according to any one of the previous claims, wherein the oil component different from the emollients constituting the polar oil component and chosen from plant origin is selected from the group consisting of *simmondsia chinensis seed oil, macademia ternifolia seed oil, ricinus communis seed oil,* and mixtures thereof.

6. Electrostatically sprayable cosmetic formulation according to any one of the previous claims, wherein the glycolipid surfactant is a rhamnolipid.

7. Electrostatically sprayable cosmetic formulation according to any one of claims 2 to 6, wherein the moisture agent is selected from the group consisting of aloe barbadensis, aloe-barbadensis extract, aloe barbadensis gel and mixtures thereof.

8. Electrostatically sprayable cosmetic formulation according to any one of claims 3 to 7, wherein the formulation comprises at least tocopherol as cosmetic additive.

9. Electrostatically sprayable skin care formulation according to any one of the previous claims, wherein the water content present in the formulation is not higher than 5 wt.-%, based on the total weight of the formulation.

10. Electrostatically sprayable cosmetic formulation according to any one of the previous claims, wherein the formulation does not comprise a propellant.

11. Electrostatically sprayable cosmetic formulation according to any one of the previous claims, wherein the cosmetic formulation is a lotion.

12. Electrostatic sprayable cosmetic formulation according to any one of the previous claims, wherein the cosmetic formulation comprises

a) 15 to 25 wt.-% ethanol;
b) 52 to 65 wt.-% of a mixture of emollients constituting the polar oil component and wherein each emollient is liquid at 20 °C and have a log $K_{ow}$ of 2 to 10;
c) 10 to 20 wt.-% of a mixture of oil components of plant origin, which differ from the emollients, constituting the further oil component;
d) 0.6 to 2.0 wt.-% of a glycolipid surfactant;
e) 0.5 to 1.5 wt.-% of at least one moisture agent;
e) 0.2 to 3 wt.-% of at least one cosmetic additive; and
f) less than 5.0 wt.-% of water;

based on the total weight of the formulation, and wherein the sum of the weight of all components present in the cosmetic formulation is 100 %.

13. Use of a glycolipid surfactant in a cosmetic formulation for facilitating the electrostatic sprayability of the cosmetic formulation comprising

a) 10 to 30 wt.-% ethanol;
b) 50 to 80 wt.-% of at least one polar oil component chosen from emollients, which are liquid at 20 °C and have a log $K_{ow}$ of 2 to 10;
c) 5 to 30 wt.-% of a further oil component different from the emollients constituting the polar oil component and chosen from oils of plant origin;

based on the total weight of the formulation, and wherein the sum of the weight of all components present in the cosmetic formulation is 100 %.

14. Use according to claim 13, wherein the glycolipid surfactant is used in an amount of 0.5 to 3 wt.-%, based on the total weight of the formulation.

15. Use according to claim 13 or 14, wherein the glycolipid surfactant is a rhamnolipid.

Figure 1

Figure 2

Figure 3

Europäisches
Patentamt

European
Patent Office

Office européen
des brevets

# EUROPEAN SEARCH REPORT

Application Number

EP 21 17 9033

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 3 795 137 A1 (IONIQ SKINCARE GMBH & CO KG [DE]) 24 March 2021 (2021-03-24)<br>* table 2 *<br>* paragraph [0034] *<br>* paragraphs [0012], [0013] *<br>----- | 1-15 | INV.<br>A61K8/04<br>A61K8/34<br>A61K8/37<br>A61K8/60<br>A61K8/92<br>A61Q19/00<br>B05D1/00 |
| A | US 2019/053602 A1 (AMARI NAOMI [JP] ET AL) 21 February 2019 (2019-02-21)<br>* paragraph [0008] *<br>* paragraph [0053] *<br>* Composition 7; table 5 *<br>----- | 1-15 | |
| A | US 2019/343731 A1 (AMARI NAOMI [JP] ET AL) 14 November 2019 (2019-11-14)<br>* table 1 *<br>----- | 1-15 | |

|  |
|---|
| TECHNICAL FIELDS SEARCHED (IPC) |
| A61K<br>C23D<br>A61Q<br>B05D |

The present search report has been drawn up for all claims

| Place of search | Date of completion of the search | Examiner |
|---|---|---|
| Munich | 30 November 2021 | Tullberg, Erik |

EPO FORM 1503 03.82 (P04C01)

**ANNEX TO THE EUROPEAN SEARCH REPORT
ON EUROPEAN PATENT APPLICATION NO.**
EP 21 17 9033

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2021

| Patent document cited in search report | | Publication date | Patent family member(s) | | Publication date |
|---|---|---|---|---|---|
| EP 3795137 | A1 | 24-03-2021 | EP | 3795137 A1 | 24-03-2021 |
| | | | WO | 2021052779 A1 | 25-03-2021 |
| US 2019053602 | A1 | 21-02-2019 | AU | 2016340484 A1 | 10-05-2018 |
| | | | BR | 112018007933 A2 | 30-10-2018 |
| | | | CA | 3001810 A1 | 27-04-2017 |
| | | | CN | 108135789 A | 08-06-2018 |
| | | | EP | 3366270 A1 | 29-08-2018 |
| | | | JP | 6203362 B2 | 27-09-2017 |
| | | | JP | 6840054 B2 | 10-03-2021 |
| | | | JP | 2017078062 A | 27-04-2017 |
| | | | JP | 2018024681 A | 15-02-2018 |
| | | | KR | 20180051670 A | 16-05-2018 |
| | | | SG | 11201803180P A | 30-05-2018 |
| | | | TW | 201729785 A | 01-09-2017 |
| | | | US | 2019053602 A1 | 21-02-2019 |
| | | | WO | 2017069079 A1 | 27-04-2017 |
| US 2019343731 | A1 | 14-11-2019 | AU | 2017388849 A1 | 11-07-2019 |
| | | | BR | 112019013464 A2 | 07-01-2020 |
| | | | CN | 110121391 A | 13-08-2019 |
| | | | CN | 112402296 A | 26-02-2021 |
| | | | EP | 3563935 A1 | 06-11-2019 |
| | | | JP | 6453434 B2 | 16-01-2019 |
| | | | JP | 2018108991 A | 12-07-2018 |
| | | | JP | 2019038856 A | 14-03-2019 |
| | | | KR | 20190078655 A | 04-07-2019 |
| | | | TW | 201841604 A | 01-12-2018 |
| | | | US | 2019343731 A1 | 14-11-2019 |
| | | | WO | 2018124227 A1 | 05-07-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9411119 A
- WO 2001012139 A
- US 20100116897 A
- DE 102017108610
- EP 3795137 A

- DE 102017108612
- DE 102017108613
- DE 102107108614
- DE 102107108615

**Non-patent literature cited in the description**

- **J. SANGSTER.** Octanol-Water Partition Coefficients: Fundamentals and Physical Chemistry. Wiley Series in solution chemistry, John Wiley & Sons, 1997, vol. 2

- **LOURITH et al.** *International Journal of Cosmetic Science,* 2009, vol. 31, 255-261